# EUROPEAN PATENT SPECIFICATION

(11) **EP 1 142 576 B1**
(45) Date of publication and mention of the grant of the patent: **02.03.2005**
(21) Application number: 98961549.7
(22) Date of filing: 25.12.1998
(51) Int. Cl.: A61K 31/557, A61P 27/06

(54) **DRUG COMPOSITIONS FOR THE TREATMENT OF OCULAR HYPERTENSION OR GLAUCOMA**
ARZNEIMITTELZUSAMMENSTELLUNG ZUR BEHANDLUNG DES ERHÖHTEN AUGENINNENDRUCKS ODER DES GLAUKOM
COMPOSITIONS DE MEDICAMENT POUR TRAITER L'HYPERTENSION OCULAIRE OU LE GLAUCOME

(43) Date of publication of application: 10.10.2001
(73) Proprietor: Sucampo AG, 6300 Zug (CH)
(72) Inventor: Ueno, Ryuji, Montgomery, MD 20854 (US)
(74) Representative: Albrecht, Thomas, Dr.
(86) International application number: PCT/JP1998/005896
(87) International publication number: WO 2000/038689

(56) References cited:
- EP-A- 0 603 800
- WO-A-97/23225
- WO-A-99/18969
- WO-A1-97/23225
- JP-A- 2 000 108
- JP-A- 6 316 525
- DIESTELHORST M ET AL: "COMPARISON OF TWO FIXED COMBINATIONS OF LATANOPROST AND TIMOLOL IN OPEN-ANGLE GLAUCOMA" GRAEFE'S ARCHIVE FOR CLINICAL AND EXPERIMENTAL OPHTHALMOLOGY, SPRINGER VERLAG, XX, vol. 236, 1998, pages 577-581, XP002905598 ISSN: 0721-832X
- DIESTELHORST M ET AL: "CLINICAL DOSE-REGIMEN STUDIES WITH LATANOPROST, A NEW OCULAR HYPOTENSIVE PGF2ALPHA ANALOGUE" SURVEY OF OPHTHALMOLOGY, SURVEY OF OPHTHALMOLOGY INC, XX, vol. 41, no. SUPPL 2, February 1997 (1997-02), pages S77-S81, XP002907101 ISSN: 0039-6257

## Description

### FIELD OF THE INVENTION

The present invention relates to the use of (a) 15-keto prostaglandin compounds and (b) FP receptor agonist type prostaglandin compounds for the manufacture of a pharmaceutical composition for treating ocular hypertension or glaucoma. Particularly, the invention relates to compositions which can enhance the effect of known compounds synergistically and can reduce the occurrence of unfavorable side effects.

### RELATED ART

Among prostaglandin compounds (hereinafter referred as "PG" or "PGs") having 20 carbon atoms in their basic structure and being called "eicosanoids", PGF₂α and PGF₂α isopropyl ester have been known to have an ocular hypotensive activity (Bahram Resul and Johan Stjernschantz "Structure-Activity Relationships of Prostaglandin Analogues as Ocular Hypotensive Agents" Cardiovascular & Renal-Overview, 781-795 1993, Current Drugs Ltd.; Carl B. Camras *et al*., *Survey of Ophthalmology* Vol.41 [Suppl 2], S61-S68, 1997, the disclosures of all of these prior arts are herein expressly incorporated by reference). These compounds have been tried in the treatment of glaucoma and ocular hypertension. However, because of their side effects, including a transient increase in intraocular pressure preceding their ocular hypotensive effect, and strong hyperemia of conjunctiva and ocular stimulation, they have not been applied for clinical use so far. Generally, it has been believed that the biological and pharmacological effect of a PG is expressed via a receptor specific to the PG. PGF₂α and PGF₂α isopropyl ester are known to have high affinity for the FP receptor, i.e. they act as FP receptor agonists (Bahram Resul *et al., Survey of Ophthalmology* Vol.41 [Suppl 2], S47-S52, 1997. In addition, a novel prostaglandin compound which acts as a FP-receptor agonist has also been reported (M. Hellberg *et al*. *IOVS* vol. 39 No. 4, 1961 March 15, 1998.

Recently, Latanoprost (general name), a FP-receptor agonist type PG compound which has a high affinity for the FP receptor, has been developed during the study of the ocular hypotensive effect of PGF₂α isopropylester. Latanoprost has a basic structure with a different number of carbon atoms from elcosanoids. The structure of Latanoprost is (+)-isopropyl-Z-7-{(1R,2R,3R,5S)-3,5-dihydroxy-2-[(3R)-3-hydroxy-5-phenylpentyl] cyclopentyl}-5-heptenoate. This compound has been reported to have an ocular hypotensive activity (Johan Stjernschantz *et al*., *Drugs of the Future* vol. 17, No. 8, 691-704, 1992; Johan Stjernschantz *et al*., *Advances in Prostaglandin, Thromboxane*, *and Leukotriene Research,* vol. 23 513-518, 1995; EP-A-0364417 (corresponds to USP 5,296,504 and JP-A-03-501025). However, the desired effect of Latanoprost has not been segregated from hyperemia of conjunctiva, an adverse side effect, and other side effects including iris pigmentation, uveitis and macula edema have also been reported (Goran Selen *et al*., *Survey of Ophthalmology* vol. 41, [Suppl 2], S125-S128, 1997; Perj Wistrand *et al*., *Survey of Ophthalmology* vol. 41, [Suppl 2], S129-S138, 1997; Jonathan A. Rowe *et al*. *American Journal of Ophthalmology* vol. 124, No. 5 683-685, 1997; Ronald E. Warwar *et al*., *Ophthalmology* vol. 105, No. 2, 263-268, 1998). Further, it was reported recently that the FP receptor might play a significant role in iris pigmentation(Tadashi Nakajima *et al*., *Abstract of The 6th Meeting of Japanese Glaucoma Society,* 136, 1995).

On the other hand, there are some non-FP receptor agonist type PG compounds, such as compounds having high affinity for the DP receptor or no substantial affinity for conventional PG receptors, known to have an ocular hypotensive activity (Carl B. Camras *et al*., Id). Compounds which do not have substantial affinity for conventional PG receptors include isopropyl unoprostone (general name)(Yasumasa Goh *et al. Japanese Journal of Ophthalmology* vol. 38, 236-245, 1994). The structure of isopropyl unoprostone is (+)-isopropyl-Z-7-[(1R,2R,3R,5S)-3,5-dihydroxy-2-(3-oxodecyl)cyclopentyl] hept-5-enoate and is classified as a docosanoid having 22 carbon atoms in its basic structure. This compound is also known as one of PG metabolites since it has an oxo-group in place of hydroxy group on the carbon atom of position 15 (so called "15-keto" type) and the carbon atoms of the positions 13 and 14 are linked through a carbon-carbon single bond(so called "13,14-dihydro" type). The fact that this compound has an ocular hypotensive activity was described in EP-A-0308135 (corresponding to USP 5,001,153 and JP-A-02-108).

The prior art does not describe nor suggest the combination of a FP receptor agonist type PG compound and a non-FP receptor agonist type PG compound, or any synergistic effects between these compounds. There is no teachings that the combination may reduce adverse side effects.

### SUMMARY OF THE INVENTION

The present invention relates to the use of (a) 15-keto-prostaglandin compounds and (b) FP receptor agonist type prostaglandin compounds for the manufacture of a composition for the treatment of ocular hypertension or glaucoma.

During an extensive study about possibility. of improving the effect by the combination of non-FP receptor agonist type PGs and a variety of compounds, the inventor surprisingly found that the effect can be synergistically enhanced by the combination of a non-FP receptor agonist type 15-keto prostaglandin compound and a FP receptor agonist type PG compound, and that adverse side effects can be reduced.

Accordingly, the present invention provides a use of (a) a non-FP receptor agonist type 15-keto-prostaglandin compound, and
(b) a FP receptor agonist type prostaglandin compound, with the proviso that when (a) is isopropyl unoprostone, (b) is not latanoprost for preparing a pharmaceutical composition for the treatment of ocular hypertension or glaucoma.

The compound used for component (a) in the present invention is a non-FP receptor agonist type 15-keto PG compound insofar as it does not have substantial affinity for the FP receptor and does not act as a FP receptor agonist. It includes, for example, a compound having high affinity for the DP receptor or a compound which does not have substantial affinity for conventional PG receptors, with a compound that does not have substantial affinity for conventional PG receptors being preferable. Examples of the compound having high affinity for the DP receptor are PGD₂, BW245C, etc. The compound that does not have substantial affinity for conventional PG receptors is selected among 15-keto-PG compounds which are so-called metabolic type PG-compounds, preferably, 13,14-dihydro-15-keto-PGs, for example, isopropyl unoprostone.

The compound used for component (b) in the present invention may be any FP receptor agonist type PG compound insofar as it has high affinity for the FP receptor and acts as a FP receptor agonist, preferably the PG compounds which can have affinity for the FP receptor as described in the above cited prior arts, especially PGF₂α, PGF₂α isopropyl ester, Latanoprost, AL6221, AL5848, AGN-191129 and AGN-191910 may be preferably used as component(b).

The present invention may be applied in relation to the treatment of various diseases and conditions in which reduction of intraocular pressure is desired, for example, glaucoma, ocular hypertension and the other diseases accompanied by increased intraocular pressure. The term "treatment or treating" in this specification and claims refers to any means of control, including preventing or curing the disease, relieving or alleviating the condition, and arresting the development of the condition.

In the present invention, the term "a subject in need of such treatment" means a subject who is suffering from a disease in which a reduction in his/her intraocular pressure is desirable, for example, glaucoma and ocular hypertension, or a subject who is susceptible to suffering from such disease as discussed above.

According to the present invention, the combination of the components (a) and (b) provides a synergistic effect. That is, since the combination enhances the desired effects of the components (a) and (b) synergistically, the dose of the each component can be reduced and, therefore, adverse side effects can be effectively reduced.

According to the present invention, an ophthalmic composition comprising the components (a) and (b) can be administrated.

In the present composition, the ratio of (a):(b) is not limited but in general about 1:0.005-1:2, preferably, about 1:0.01-1.

The dose of each component in the present invention should be determined by a doctor according to, such as, the state and severity of the condition to be treated, the object of treatment and total amount of the administration. Typically, the amount of the component (a) in the composition used for topical administration is about 0.005-2 wt%, preferably, 0.01-1 wt%. The amount of the component (b) in the composition used for topical administration is about 0.0001-2 wt%, preferably, 0.0005-1 wt%. The components (a) and (b) may be administered either simultaneously or individually.

The composition of the present invention may be formulated to contain both of the components (a) and (b) in a single dosage unit, or may be a package consisting of separate dosage units containing each component respectively. The composition of the present invention may further contain any conventional compound used in ophthalmologic field such as carrier and adjuvant.

The composition of the present invention may be formulated as liquids such as solution, emulsion, and suspension, or as semisolids such as gel or ophthalmic ointment.

Examples of diluents for an aqueous solution or suspension include distilled water and physiological saline. Examples of diluents for a non-aqueous solution or suspension include vegetable oil, liquid paraffin, mineral oil, propylene glycol and p-octyldodecanol and the like. In addition, isotonic agents, such as sodium chloride, boric acid and sodium citrate to make isotonic with the lacrimal fluid, and buffering agents, such as borate buffer and phosphate buffer to maintain pH about 5.0 to 8.0 may be contained in the present composition. Further, stabilizing agents such as sodium sulfite and propylene glycol, cheating agents such as sodium edetate, thickening agents such as glycerin, carboxymethyl cellulose and carboxyvinyl polymer, and preservatives such as methyl paraben, propyl paraben may be contained in the composition. The ingredients are sterilized by means of, for example, passing through a bacterial filter or heating.

The ophthalmic ointment may contain, such as, vaseline, Zelen 50, Plastibase and Macrogol as base components and a surfactant for increasing hydrophilicity. It may also contain gelling agents such as carboxymethylcellulose, methylcellulose, carboxyvinyl polymer, as well.

In addition, the composition may contain antibiotics such as chloramphenicol and penicillin.

### BRIEF DESCRIPTION OF THE DRAWINGS

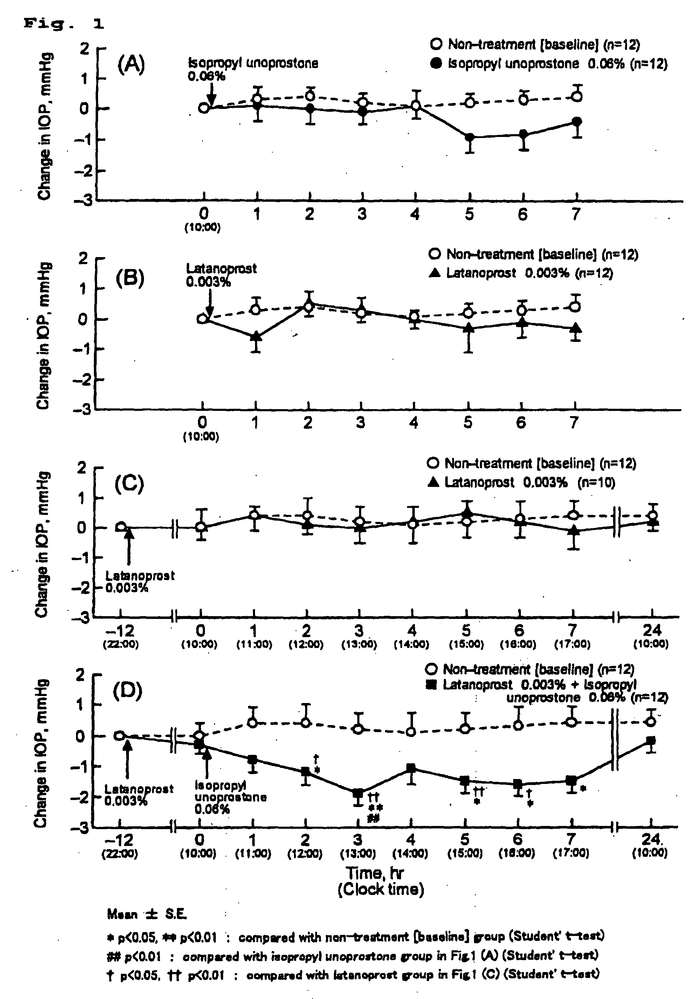

Figure 1 (A) shows a time course of intraocular pressure followed by a single instillation of 0.06 % isopropyl unoprostone.
Figure 1(B) shows a time course of intraocular pressure followed by a single instillation of 0.003 % Latanoprost at 10 o'clock.
Figure 1(C) shows a time course of intraocular pressure followed by a single instillation of 0.003% Latanoprost at 22 o'clock.
Figure 1(D) shows a time course of intraocular pressure followed by the combined instillation of 0.003% Latanoprost at 22 o'clock and 0,06% isopropyl unoprostone at a time 12 hours after that.

The present invention will be illustrated in more detail by the following examples.

### Test example

### 1. Test Substance (Not covered by the scope of the claims).

In order to study the ocular hypotensive effect of the combined administration of isopropyl unoprostone and Latanoprost, 0.06% isopropyl unoprostone eye drops and 0.003% Latanoprost eye drops were prepared separately. Each concentration of the active ingredients is about a half of conventional clinical composition.

### 2. Animal

Male normal cynomolgus monkeys (body weight of 4.5-6.5 kg) having good health and fore eye condition were used.

### 3. Administration schedule (Not covered by the scope of the claims).

Each of isopropyl unoprostone and Latanoprost eye drops was administrated topically to the respective monkeys by means of micro pipette (Gilson, U.S.A) in an amount of 30µl per eye at 10 o'clock. For clinical use, Latanoprost was directed to administrate once a day at night, and it was reported from an examination with normal volunteers that the maximum decrease of intraocular pressure occurred about 12 hours after the administration. Therefore, in this example, the effect of Latanoprost eye drops on intra ocular pressure when administrated at 22 o'clock were also determined. For the combination eye drop of isopropyl unoprostone and Latanoprost, Latanoprost eye drops were administrated at 22 o'clock and then isopropyl unoprostone were administrated at 10 o'clock in the next morning, that is 12 hours after Latanoprost administration.

### 4. Measurement of Intraocular Pressure

The monkeys were anesthetized intramuscularly with 5.0-7.5 mg/kg of Ketamin (Sankyo Pharmaceuticals Co. Ltd.). A time course of the intraocular pressure was determined under topical anesthetization by dropping 0.4 % oxybuprocaine hydrochloride to eyes (Santen Seiyaku), by means of applanation pneumatonograph (Nippon Alcon).

### 5. Results

Changes in intraocular pressures (Δ IOP) of each administration group compared with the pressure determined before the administration and are shown in Fig. 1(A)-(D).

Single ocular administration of 0.06 % isopropyl unoprostone did not show any significant change in IOP when compared with a control (non-treatment) group (Fig. 1(A)).

Single ocular administration of 0.003% Latanoprost at 10 o'clock(Fig. 1(B)) nor at 22 o'clock (Fig. 1(C)) did not show any significant change in IOP when compared with the control(non-treatment) group.

On the contrary, when 0.003% Latanoprost was administrated at 22 o'clock and 0.06 % isopropyl unoprostone was administrated at 10 o'clock the next morning, that is 12 hours after Latanoprost administration(combined administration), the combination showed a significant decrease of IOP compared with any one of the groups of control(non-treatment), single administration of isopropyl unoprostone, and single administration of Latanoprost (Fig. 1 (D)).

From the above results, it is demonstrated that the combined administration of isopropyl unoprostone and Latanoprost decreases intraocular pressure synergistically. (the use of this specific combination is not covered by the scope of the claims).

From the above results, it can be said that a synergistic ocular hypotensive effect will be obtained from the combination of the components (a) and (b).

### Industrial Applicability

The pharmaceutical composition of the present invention is useful in relation to the treatment of ocular hypertension and glaucoma.

## Claims

1. Use of (a) prostaglandin compound which does not have substantial affinity for conventional prostaglandin receptors said compound being a 15-keto prostaglandin compound and (b) a FP receptor agonist type prostaglandin compound in the manufacture of a pharmaceutical composition for the treatment of ocular hypertension or glaucoma with the proviso that when (a) is isopropyl unoprostone, (b) is not latanoprost.

2. The use of Claim 1, wherein said (a) prostaglandin compound which does not have substantial affinity for conventional prostaglandin receptors and said (b) FP agonist type prostaglandin compound are formulated in a single dosage unit.

3. The use of Claim 1, wherein said (a) prostaglandin compound which does not have substantial affinity for conventional prostaglandin receptors and said (b) FP receptor agonist type prostaglandin compound are formulated in separate dosage units respectively.

4. The use of Claim 1, wherein said prostaglandin compound which does not have substantial affinity for conventional prostaglandin receptors is a 13,14-dihydro-15-keto-prostaglandin compound.

5. The use of Claim 1, wherein said prostaglandin compound which does not have substantial affinity for conventional prostaglandin receptors is isopropyl unoprostone.

6. The use of any one of claims 1 to 5 wherein the FP receptor agonist type prostaglandin compound is selected from the group consisting of PGF₂α, PGF₂α isopropylester, Latanoprost, AL 6221, AL 5848, AGN-191129 and AGN-191910, with the proviso that when (a) is isopropyl unoprostone, (b) is not latanoprost.

7. The use of Claim 6, wherein said FP receptor agonist type prostaglandin compound is Latanoprost.

## Patentansprüche

1. Verwendung (a) einer Prostaglandin-Verbindung, die keine wesentliche Affinität für herkömmliche Prostaglandin-Rezeptoren hat, wobei die Verbindung eine 15-Ketoprostaglandin-Verbindung ist und (b) einer Prostaglandin-Verbindung vom FP-Rezeptor-Agonist-Typ bei der Herstellung einer pharmazeutischen Zusammensetzung für die Behandlung von erhöhtem Augeninnendruck oder Glaukom mit der Maßgabe, dass, wenn (a) Isopropylunoproston ist, (b) nicht Latanoprost ist.

2. Verwendung nach Anspruch 1, wobei die (a) Prostaglandin-Verbindung, die keine wesentliche Affinität für herkömmliche Prostaglandin-Rezeptoren hat und die (b) Prostaglandin-Verbindung vom FP-Rezeptor-Agonist-Typ in einer Einzeldosiseinheit formuliert sind.

3. Verwendung nach Anspruch 1, wobei die (a) Prostaglandin-Verbindung, die keine wesentliche Affinität für herkömmliche Prostaglandin-Rezeptoren hat und die (b) Prostaglandin-Verbindung vom FP-Rezeptor-Agonist-Typ jeweils in getrennten Dosiseinheiten formuliert sind.

4. Verwendung nach Anspruch 1, wobei die Prostaglandin-Verbindung, die keine wesentliche Affinität für herkömmliche Prostaglandin-Rezeptoren hat, eine 13,14-Dihydro-15-ketoprostaglandin-Verbindung ist.

5. Verwendung nach Anspruch 1, wobei die Prostaglandin-Verbindung, die keine wesentliche Affinität für herkömmliche Prostaglandin-Rezeptoren hat, Isopropylunoproston ist.

6. Verwendung nach einem der Ansprüche 1 bis 5, wobei die Prostaglandin-Verbindung vom FP-Rezeptor-Agonist-Typ ausgewählt ist aus der Gruppe bestehend aus PGF₂α, PGF₂α-Isopropylester, Latanoprost, AL 6221, AL 5848, AGN-191129 und AGN-191910, mit der Maßgabe, dass, wenn (a) Isopropylunoproston ist, (b) nicht Latanoprost ist.

7. Verwendung nach Anspruch 6, wobei die Prostaglandin-Verbindung vom FP-Rezeptor-Agonist-Typ Latanoprost ist.

## Revendications

1. Utilisation d'un composé de prostaglandine (a) qui n'a pas d'affinité réelle pour les récepteurs de la prostaglandine conventionnels, ledit composé étant un composé de la 15-céto-prostaglandine et un composé de prostaglandine de type agoniste des récepteurs FP (b) dans la fabrication d'une composition pharmaceutique pour le traitement de l'hypertension oculaire ou du glaucome à condition que (a) soit l'unoprostone isopropyle, (b) ne soit pas le latanoprost.

2. Utilisation de la revendication 1, dans laquelle ledit composé de prostaglandine (a) qui n'a pas d'affinité réelle pour les récepteurs de la prostaglandine conventionnels et ledit composé de prostaglandine de type agoniste des récepteurs FP (b) sont formulés en dose unique.

3. Utilisation de la revendication 1, dans laquelle ledit composé de prostaglandine (a) qui n'a pas d'affinité réelle pour les récepteurs de la prostaglandine conventionnels et ledit composé de la prostaglandine de type agoniste des récepteurs FP (b) sont formulés respectivement en doses séparées.

4. Utilisation de la revendication 1, dans laquelle ledit composé de prostaglandine qui n'a pas d'affinité réelle pour les récepteurs de la prostaglandine conventionnels, est un composé de 13,14-dihydro-15-céto-prostaglandine.

5. Utilisation de la revendication 1, dans laquelle ledit composé de prostaglandine qui n'a pas d'affinité réelle pour les récepteurs de la prostaglandine conventionnels est l'unoprostone isopropyle.

6. Utilisation de l'une quelconque des revendications 1 à 5 dans laquelle le composé de prostaglandine de type agoniste des récepteurs FP est choisi parmi le groupe constitué de PGF₂α, d'isopropylester de PGF₂α, de latanoprost, d'AL 6221, d'AL 5848, d'AGN-191129 et d'AGN-191910, à condition que lorsque (a) est l'unoprostone isopropyle, (b) n'est pas le latanoprost.

7. Utilisation de la revendication 6, dans laquelle ledit composé de prostaglandine de type agoniste des récepteurs FP est le latanoprost.
